# EUROPEAN PATENT APPLICATION

(11) **EP 0 637 448 A1**
(43) Date of publication of application: **08.02.1995**
(21) Application number: 94202075.1
(22) Date of filing: 16.07.1994
(51) Int. Cl.: A61K 31/16

(54) **Combinations of glycinamide or N-acetylglycinamide with analgesic compounds**

(30) Priority: 19.07.1993 IT MI931586
(71) Applicant: ISCOFAR SAS DI PAOLO E. GHIRARDI, I-20129 Milano (IT)
(72) Inventor: Ghirardi, Paolo c/o Iscofar, I-20129 Milano (IT)
(74) Representative: Dragotti, Gianfranco

(57) **Abstract**

Glycinamide and the N-acetyl derivative thereof possess analgesic and antiinflammatory activity.

These two substances enhance in considerable amount the activity of well-known active principles of analgesic non-narcotic and antiinflammatory non-steroidal type.

## Description

The present invention concerns pharmaceutical compositions with analgesic, antiphlogistic and antirheumatic activity and, particularly, the use of glycine derivatives in combination with analgesic, non-narcotic or non-steroidal antiinflammatory drugs.

To relieve both acute and chronic pain, a wide range of drugs capable of reducing or resolving this symptom is presently available: narcotic analgesics, non-narcotic analgesics active on the central system, local anaesthetics, anticephalea and non-steroidal antiinflammatory analgesic drugs.

The narcotic analgesics are normally employed in the therapy of oncological or anyhow relentless pain. The non-narcotic analgesics active on the central system are carefully used because of a number of side effects.

The anticephalea drugs are solely indicated in some cephalea forms.

The non-steroidal antiinflammatory drugs (NSAIDs) represent nowadays the first choice drugs owing to the peculiar pharmacological profile that highlighted their multidirectional analgesic mechanism.

The NSAIDs develop very useful effects by virtue of their multiple action mechanisms: inhibition of prostaglandin synthesis, inhibition of lysosomial enzymes, inhibition of chemotaxis, inhibition of complement activation, inhibition of free radicals formation (for some of them).

It cannot be lastly forgotten that, on inflammatory pains, typical of arthroreumatic diseases, the FANS, because of their powerful antiinflammatory action, produce a twofold antalgic effect: antiphlogistic and visceral-peripheral analgesic.

Some unfavourable aspects are however associated with the pharmacological properties, such as lesive effects on the gastrointestinal system, a limited action time (this last obstacle being only apparently overcome with the parenteral use which, by the way, is not usually feasible).

Glycine is a natural amminoacid that until now has been used in the technique of pharmaceutical preparations as excipient, especially in the case of active principles dosed in very small quantities or in the case of lyophilized preparations.

From the activity point of view, however, only a behaviour as inhibiting neurotransmitter at the spinal cord level has been noticed for this aminoacid.

It is therefore evident that both glycine and derivatives thereof have not been of interest until now from the therapeutical point of view: among these derivatives glycinamide and its acetyl derivative are to be mentioned.

Recent researches carried out by the inventors evidenced surprisingly that, by means of the classical test carried out on the mouse to find out molecules with analgesic visceral or peripheral action (writhing test), the glycinamide and the acetyl derivative thereof develop a NSAIDs-like activity, such as to raise remarkable interest if it is considered that these compounds are extremely well tolerated (LD₅₀ in the mouse orally higher than 2 g/Kg).

It has also been found and it is an object of the present invention that the accompanying presence of glycinamide or of the acetyl derivative thereof in pharmaceutical compositions containing as the acive principle an analgesic, non narcotic or a non-steroidal antiinflammatory drug, selected among those well-known, enhances its activity in much greater proportion compared with that to be expected on the basis of the properties of each component.

Otherwise stated, it has been found that, the therapeutical effect being the same, the presence in the pharmaceutical composition of glycinamide or of the acetyl derivative thereof permits the content of the main active principle to be reduced to one half or less with respect to the doses normally used to obtain such a therapeutical effect. In order to experimentally test the above analgesic effect, which involves the µ and K type opiate receptors, the writhing test (literally: "induced abdominal contracture test") with 2 mg/mg of phenylquinone or paraphenylbenzoquinone (0.02% in 5% ethanol), has been choosed.

Since phenylquinone is a photosensitive, said solution was kept dark bottle at 37^{o}C with continuous stirring.

Groups of animals were treated orally with different doses of glycinamide at various time intervals before phenylquinone being administered by intraperitoneal way.

For each experimental point 3 groups of 10 animals each were tested.

After the phenylquinone administration, the animals were placed in glass cylinders and the abdominal contractions were recorded for 20 minutes starting form the fifth minute following the intraperitoneal administration of phenylquinone. As an average, about 50±15 abdominal contractions were recorded during the surveying time.

The choice of phenylquinone as the writhing inductor is justified by the greater qualitative constance and higher number of contractions obtainable when compared with other inductors such as acetylcholine, acetic acid, ethacrynic acid, air, etc.

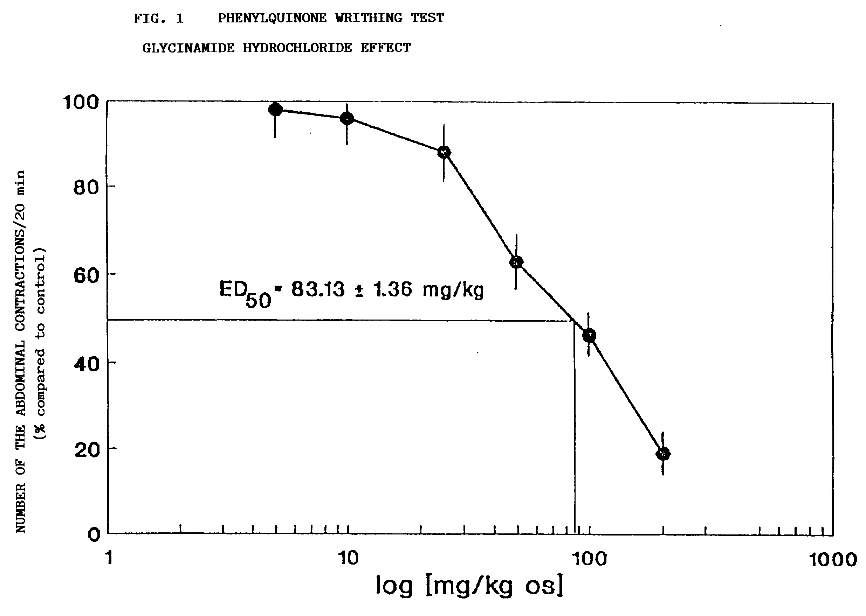

Glycinamide, always used as hydrochloride, shows (fig. 1) a noticeable anti-writhing action that reaches its peak, with a 81.3% reduction, at the orally administered dose of 200 mg/kg.

The ED₅₀ has been estimated in the order of 82.86±1.43 mg/kg.

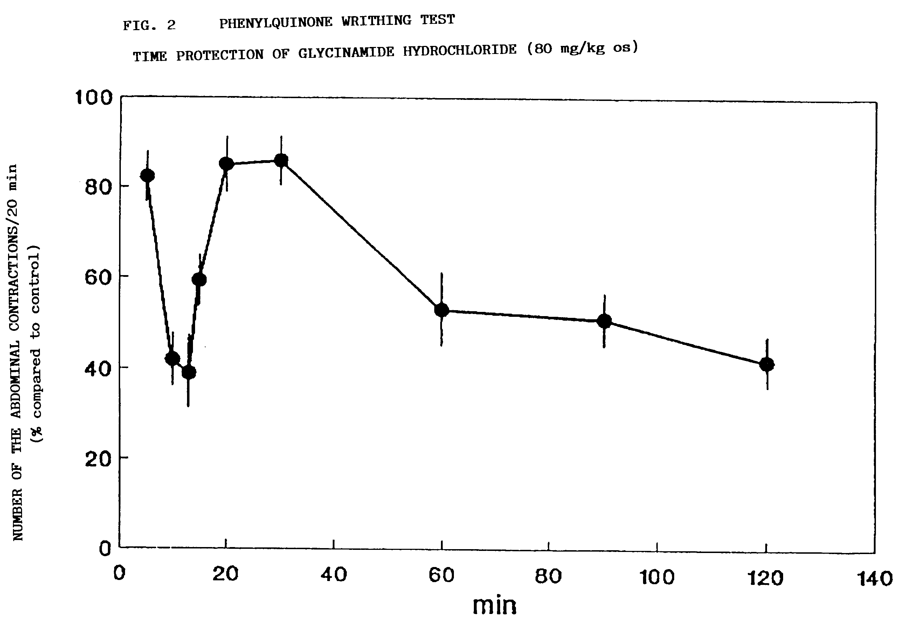

This concentration demonstrated that the maximum analgesic effect is shown about 13 minutes after the administration (fig. 2)
As typical arylalcanoic FANS of the phenylacetic group, sodium diclofenac has been selected since it can inhibit the phenylquinone induced writhing in a dose-dependent manner.

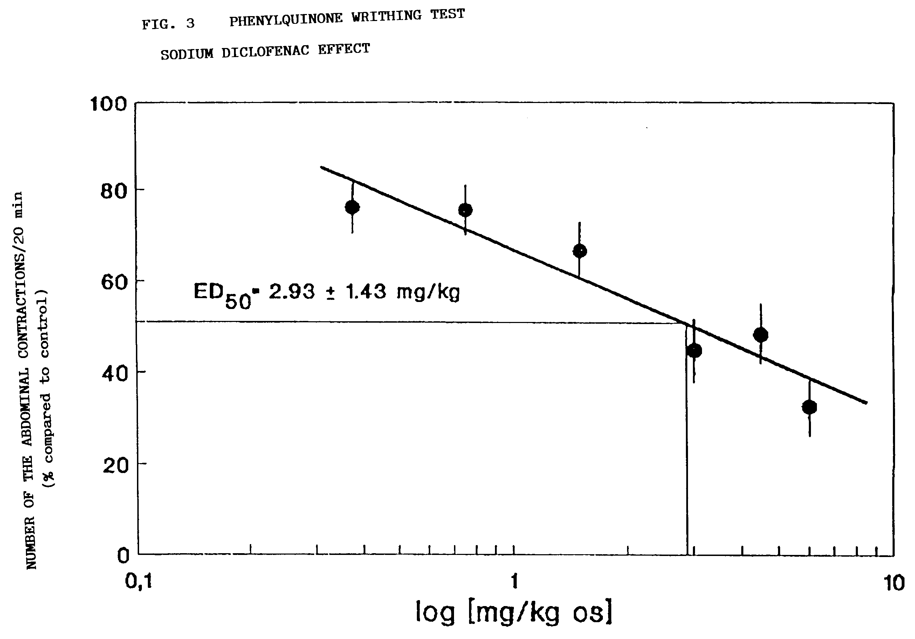

Its ED₅₀ is equal to 2.93±1.43 mg/kg (fig. 3).

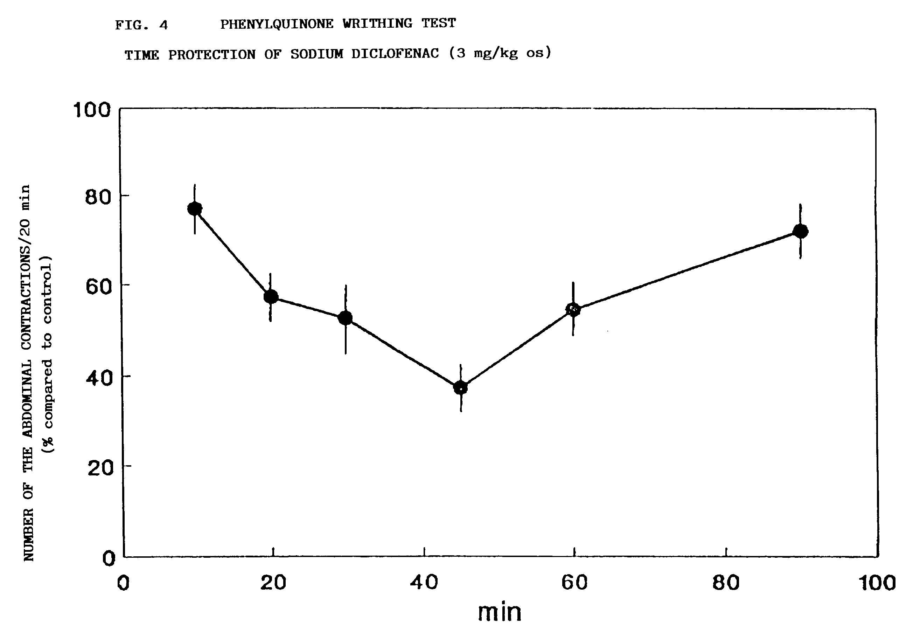

The adiministering of this dose as a time function has shown that the maximum effect is achieved 45 minutes after the oral administration (fig. 4).

The glycinamide, administered 13 minutes before testing, increases significantly (P=0.041) the anti-writhing action of sodium diclofenac yet at the 15 mg/kg dose (Table 1).

Said action reaches its peak at the 25 mg/kg glycinamide concentration: in such experimental point, the ED₅₀ of the antiphlogistic drops to 1.12±1.2 mg/kg.

Glycinamide, orally administered to the mouse at the dose of 50 mg/kg, which does not develop an anti-edema effect (50 minutes before injection of 0.1 ml of 1% carrageenin aqueous solution under the plantar surface of a hind leg), enhances always in signifcant way the anti-edema action of sodium diclophenac, indomethacin, ibuprofen, and salicylic acid, orally administered at the effective dose one hour before the carrageenin edema test (Table 2).

In other words, based on writhing test, 25 mg/kg of glycinamide (equal to 1.75 g in the adult man) enhance the antiphlogistic and visceral peripheral analgesic effect of sodium diclofenac by at least 2.5 times and similarly, based on the carrageenin edema test, 50 mg/kg (equal to about 3.5 g in the adul man), enhance the effect of sodium diclofenac by at least 2.8 times.

All things considered, the therapeutic effect which is observed in man with 50 mg of sodium diclofenac (one normal tablet) can be obtained with only 20 mg in the presence of 1.75 g of the glycine derivative.

**TABLE 1**

| EFFECT OF GLYCINAMIDE HCl ON THE ANTIWRITHING ACTION OF SODIUM DICLOFENAC | | |
|---|---|---|
| | ED₅₀ ± D.5 (mg/kg orally) | P |
| Glycinamide | 82.86±1.43 | -- |
| Sodium diclofenac | 2.93±1.38 | -- |
| + Glycinamide HCl 15 | 1.86±1.15 | 0.041 * |
| + Clycinamide HCl 25 | 1.12±1.2 | 0.000 ** |
| + Clycinamide HCl 50 | 1.05±1.31 | 0.002 * |
| + Glycinamide HCl 75 | 1.02±1.26 | 0.002 * |
| + Glycinamide HCl 100 | 1.28±1.16 | 0.003 * |
| The abdominal writhing has been induced by the intraperitoneal administration in mouse of phenylquinone (2 mg/kg) 5 minutes before testing. This involved recording the number of contractions which occurred during 20 minutes observation. Glycinamide hydrochloride (mg/kg) was orally administered 13 minutes before testing Sodium diclofenac orally 15 minutes before testing at the following dosages: 0.375; 1.5; 3.0; 4.5 and 6.0 mg/kg. ED₅₀ = calculated by the Finney method (1971). S.D. = sample standard deviation. P = probability level of the two tail Student test function "t" | | |

| | | |
|---|---|---|
| (* = P < 0.05; ** = P < 0.01). | | |

**TABLE 2**

| EFFECT OF THE GLYCINAMIDE HCl ON THE ANTI-INFLAMMATORY ACTION OF SOME NSAIDs ON THE EDEMA FROM CARRAGEENIN | | |
|---|---|---|
| | ED₅₀ ± S.D. (mg/kg per os) | |
| | ― | + Glycinamide HCl 50 |
| Sodium diclofenac | 10 ± 3.6 | 3.6 ± 1.80 ** |
| Indomethacin | 84 ± 12 | 72 ± 8.00 ** |
| Ibuprofen | 20 ± 2.4 | 16 ± 2.16 ** |
| Acetylsalicylic acid | 5.1 ± 0.8 | 3.9 ± 1.00 ** |
| Glycinamide HCl | n.d. | ----------- |
| The edema has been induced by injecting under the plantar surface of a mouse leg 0.1 mg of a 1% aqueous solution of carrageenin. Glycinamide hydrochloride and each FANS (mg/kg) were orally administered 50 and 60 minutes respectively before the carrageenin subplantar injection. The volume of the acute edema was determined by means of pletmometer as differential value between the recordals made at the time 0 and after 3 hours. At least 4 groups of 8 animals were tested for each substance. ED₅₀ = dose reducing edema by 50%, calculated by Finney method (1971). S.D. = sample standard deviation. P = probability level of Student test function "t" | | |

| | | |
|---|---|---|
| (**P = < 0.01). | | |

Two properties resulted consequently to be surprising:
a) the peripheral analgesic activity of glycinamide, shared both qualitatively and quantitatively by the acetyl derivative thereof as well.
b) the enhancing property for non-narcotic analgesic drugs (codeine, phenyramidol hydrochloride, lefetamine, methotrimeprazine, nefompam hydrochloride, dextro and levopropoxyphene, viminol hydroxybenzoate) and NSAIDs (e.g., salicylates and derivatives of N-phenylantranilic acid, pyrazolone and oxicam derivatives, indole and related derivatives, phenylacetic and phenylpropionic derivatives of the arylcanoic acid, hydroxamic acid derivatives, methanesulphonanilide and para-aminophenol derivatives).

The example mentioned herein of the glycinamide-sodium diclofenac enhancement refers therefore, still for the sake of an indicative and non-limiting purpose, to the enhancement produced by the glycinamide and N-acetylglycinamide on the typical representative products of the above classes (e.g., codeine and derivatives; dextropropoxyphene and analogues; aspirin adn derivatives; fenamates and derivatives; amidopyrine, phenylbutazone; dipyrone and derivatives; piroxicam and other oxicam derivatives; indomethacin and other indenic, pyrrolic, and thiazolic analogues; etodolac and ketorolac; ibuprofen, naproxen, ketoprofen, indoprofen, flurbiprofen, tiaprofenic acid, and analogues; oxametacin and ibuproxam; nimesulide and other methanesulphonanilide derivatives; paracetamol and phenacetin).

Glycinamide and N-acetylglycinamide enhance consequently the action of peripheral analgesic drugs, a specific and still more surprising effect which glycine does not produce.

Since they also have (central nootropic) properties (increase of the cognitive ability, improvement of mood tone and alertness, said enhancement achieves two improvements in the use both of NSAIDs and of non narcotic analgesic drugs:
a) utilization of drugs of both classes at doses lower as compared with routine ones, but with enhancement or at least maintenance of the analgesic effect;
b) decrease of the undesirable NSAIDs effects, either local (gastrointestinal irritations and lesions) or central (deterioration of the cognitive functions), and of the unwanted effects of the non narcotic analgesic drugs (neurosedation).

Some formulation examples illustrating few embodiments of the present invention, without anyhow limiting it, are now provided.

In column A same examples of a combination of drugs producing an increase in the analgesic action in respect to the marketed preparations are given, whereas in that one marked with the letter B some examples of drugs causing an equianalgesic activity as compared to the marketed preparations are presented.

### Example 1 - Sodium Diclofenac: one tablet doses

| | A | B |
|---|---|---|
| Sodium diclofenac | 50 mg | 20 mg |
| Glycinamide | 1.75 g | 1.75 g |
| Excipients: lactose 20 mg; starch 40 mg; magnesium stearate 2 mg; polivinylpyrrolidone 5 mg. | | |

### - Sodium diclofenac: one suppository dose

| | A | B |
|---|---|---|
| Sodium diclofenac | 100 mg | 40 mg |
| Glycinamide | 1.75 g | 1.75 g |
| Excipients: solid semisynthetic glycerides enought to 3 g | | |

### - Sodium diclofenac: for an intramuscular vial dose

| | A | B |
|---|---|---|
| Sodium diclofenac | 75 mg | 30 mg |
| glycinamide | 1.75 g | 1.75 g |
| Excipients: propylene glycol 580 mg; benzyl alcohol 120 mg; water for injectable preparations enough to 4 ml | | |

### - Diclofenac for an eyewash (5 ml counter)

| | A | B |
|---|---|---|
| Sodium diclofenac | 5 mg | 2.5 mg |
| Glycinamide | 87.5 mg | 87.5 mg |
| Excipient glyceril-polyethyleneglycol ricinoleate 250 mg; tromethamine 30 mg; sodium merthiolate 0.2 mg; water for injectable preparation enough to 5 ml | | |

### - Diclofenac for otologic drops (5 ml counter)

| | A | B |
|---|---|---|
| Sodium diclofenac | 5 mg | 2.5 mg |
| Glycinamide | 87.5 mg | 87.5 mg |
| Procaine hydrochloride | 5 g | 5 g |
| Excipients: glycerol enough to 5 g | | |

### EXAMPLE No. 2 Ketoprofene: for one tablet dose

| | A | B |
|---|---|---|
| Ketoprofene (nucleos) | 25 mg | 12.5 mg |
| Glycinamide (coating) | 1.75 g | 1.75 g |
| Excipients: carboxymethylstarch 25 mg; polyvinilpyrrolidone 15 mg. | | |

### - Ketoprofene: for one vial dose

| | A | B |
|---|---|---|
| Ketoprofene (as sodium salt) | 100 mg | 50 mg |
| Glycinamide | 1.75 g | 1.75 g |
| Excipients: benzyl alcohol 0.0625 ml; water for injectable formulations enough to 4 ml | | |

### - Ketoprofene: intradermal gel doses

| | A | B |
|---|---|---|
| Ketoprofene | 250 mg | 125 mg |
| Glycinamide | 2 g | 2 g |
| Excipients: carboxypolymethylene 0.2 g; ethyl alcohol 4 ml; p-hydroxybenzoic acid esters 0.01 g; diethanolamine 0.135; purified water enough to 10 ml. | | |

### EXAMPLE No. 3 - Dextropropoxyphene hydrochloride: one dragée doses

| | A | B |
|---|---|---|
| Dextropropoxyphene hydrochloride | 30 mg | 15 mg |
| N-acetylglycinamide | 1.5 g | 1.5 g |
| Excipients: lactose 40 mg; starch 30 mg; talc 15 mg; magnesium carbonate 6 mg; colophony 3 mg; sucrose 60 mg. | | |

### - Dextropropoxyfene hydroclhoride: one suppository dose

| | A | B |
|---|---|---|
| D-propoxyfene hydrochloride | 75 mg | 40 mg |
| N-acetylglycinamide | 1.5 g | 1.5 g |
| Excipients: semisinthetic glycerides enough to 3 g | | |

### -Dexotropropoxyfene hydrochloride: one vial dose

| | A | B |
|---|---|---|
| D-propoxyfene hydrochloride | 75 mg | 40 mg |
| N-acetylglycinamide | 1.5 g | 1.5 g |
| Excipients: benzyl alcohol 0.02 ml; water for injectable preparations enough to 4 ml | | |

### EXAMPLE No. 4 Acetylsalicylic acid: a tablet doses

| | A | B |
|---|---|---|
| Acetylsalicylic acid | 325 mg | 165 mg |
| Glycinamide | 1.75 g | 1.75 g |
| Excipients: magnesium hydroxide 100 mg; maize starch 54 mg; talc 10 mg; sodium croscarmellose 11 mg. | | |

### Lysine acetylsalicylate; one packet doses

| | A | B |
|---|---|---|
| Lysine Acetylsalicylate | 0.9 g | 0.45 g |
| (equivalent to acetylsalicylic acid | 0.5 g | 0.25 g) |
| Glycinamide | 1.75 g | 1.75 g |
| Excipients tangerine flavor 20 mg; glycyrrhizined ammonium 3 mg. | | |

### - Acetylsalicylate of lysine: a tiny bottle sterile powder

| | A | B |
|---|---|---|
| Lysine Acetylsalicylate | 0.9 g | 0.45 g |
| (equivalent to acetylsalicyle acid | 0.5 g | 0.25 g) |
| Glycinamide | 1.75 g | 1.75 g |
| Excipients: water for injections enough to 4 ml. | | |

### EXAMPLE NO. 5 - Dipyrone as oral drops; one ml contains:

| | A | B |
|---|---|---|
| Dipyrone | 0.5 g | 0.25 g |
| Glycinamide | 1.75 g | 1.75 g |
| Excipients: sodium phosphate 5.7 mg; monosodium phosphate 5.7 mg; citrus flavor 7 mg; purified water enough to 1 ml. | | |

### EXAMPLE NO. 6 - Piroxicam: doses for one soluble tablet

| | A | B |
|---|---|---|
| Pyroxicam | 20 mg | 8 mg |
| N-acetylglycinamide | 1.5 g | 1.5 g |
| Excipients: lactose 50 mg; microcrystalline cellulose 80 mg; Hydroxypropylcellulose 35 mg; sodium stearylfumarate 5 mg. | | |

### - Piroxicam: one suppository dose

| | A | B |
|---|---|---|
| Piroxicam | 20 mg | 8 mg |
| N-acetylglycinamide | 1.5 g | 1.5 g |
| Excipients: propylgallate 0.2 mg; semisynthetic glycerides enough to 3 g | | |

### EXAMPLE 7 - Indomethacin: doses for one lyophilized vial

| | A | B |
|---|---|---|
| Indomethacin meglumine | 77.2 mg | 38.6 mg |
| (equivalent to indomethacin) | 50 mg | 25 mg) |
| Glycinamide | 1.75 g | 1.75 g |
| Excipients: monopotassium phosphate 4.78 mg; dihydrated disodium phosphate 5.54 mg; water for injection enough to 4 ml. | | |

### EXAMPLE NO. 8 - Ibuprofen: doses for one packet (granular)

| | A | B |
|---|---|---|
| Ibuprofen | 600 mg | 400 mg |
| N-acetylglycinamide | 1.5 g | 1.5 g |
| Excipients: fructose 2 g; citric acid 0.4 g; sodium citrate 0.25 g; hydropropylmethylcellulose phthalate 30 mg; propyleneglycol alginate 150 mg; sodium saccharinate 30 mg; raspberry flavor 1.2 mg. | | |

### Ibuprofen: a vaginal wash dose

| | A | B |
|---|---|---|
| Ibuprofen lysine salt | 1.4 g | 0.7 g |
| N-acetylglycinamide | 4.5 g | 4.5 g |
| Excipients: phenylethyalcohol 0.35 g: purified water enough to 140 ml. | | |

### EXAMPLE No. 9 - Naproxen: doses for one tablet

| | A | B |
|---|---|---|
| Naproxen | 500 mg | 250 mg |
| Glycinamide | 1.75 g | 1.75 g |
| Excipients: polyvinylpirrolydone 12 mg; magnesium stearate 2 mg; sodium croscarmellose 22 mg. | | |

### - Naproxen: one suppository dose

| | A | B |
|---|---|---|
| Naproxen | 500 mg | 250 mg |
| Glycinamide | 1.75 g | 1.75 g |
| Excipients: semisynthetic glycerides 3 g | | |

### EXAMPLE NO. 10 - Nimesulide: dose for one dispersible formulation

| | A | B |
|---|---|---|
| Nimesulide | 100 mg | 40 mg |
| N-acetylglycinamide | 1.5 g | 1.5 g |
| Excipients: cetomacrogol 1000 8 mg; sucrose 1 g; maltdextins 15 mg; citric acid 30 mg; orange flavor 45 mg. | | |

### EXAMPLE NO. 11 - Paracetamol: doses for one tablet

| | A | B |
|---|---|---|
| Paracetamol | 500 mg | 250 mg |
| Glycinamide | 1.75 g | 1.75 g |
| Excipients: microcrystalline cellulose 30 mg; gelatin 20 mg; talc 6 mg; magnesium stearate 5 mg; colloidal silica 1.2 mg. | | |

### - Paracetamol syrup, 100 ml contain:

| | A | B |
|---|---|---|
| Paracetamol | 2.4 g | 1 g |
| Glycinamide | 1.75 g | 1.75 g |
| Excipients: sucrose 45 g; sorbitol 5 g; propyleneglycol 10 g; alcohol 7 g; saccharin 0.2 g; sodium citrate 0.15 g; sour black cherry flavor 0.5 g; methyl p-hydroxybenzoate 0.126 g; propyl p-hydroxybenzoate 0.014 g; purified water enough to 100 ml. | | |

Sofar the pharmaceutical compositions based on glycinamide or N-acetylglycinamide are concerned, these can be realized both in the form of tablets, of effervescent type if needed, and all the other forms suitable to be orally administered (also due to the excellent water solubility of both these active principles).

The dosages of glycinamide and of the acetyl derivative thereof might be also very high considering its excellent tolerability and extremely low toxicity.

In practice, these compositions will contains as a rule at least 0.5 grams of glycinamide or N-acetylglycinamide. The glycinamide dosage is also depending on the pharmaceutical form, thus in combination with NSAIDs there are from 1.75 g for a tablet to 17.5 mg/ml for 1 ml eyewash, up to 4.5 g for 140 ml of vaginal wash (equal to 32 mg/ml).

Obviously the reference to the orally administered compositions has not a limiting purpose, since the other pharmaceutical forms that differ from those for oral administration, including the injectable, as well as intracutaneous and intramuscolar also for cosmetic use, can be equally realized and foreseen.

The compositions containing glycinamide or N-acetylglycinamide are mainly used for treating cephaleas, toothache or other not particularly intense inflammatory states: as a matter of fact, in such cases the desired therapeutical result, without the side effects anyhow related to the employment of the usual antiinflammatory and analgesic drugs, is achieved.

Of course, the traditional pharmaceutical techniques, as well as the usual carriers and excipients, will be utilized to prepare these pharmaceutical compositions.

## Claims

1. Pharmaceutical composition characterized in that it contains as active principles a well known analgesic and/or non-steroidal antiinflammatory non-narcotic drug and a compound selected between glycinamide and N-acetylglycinamide together with the usual carriers and excipients.

2. Pharmaceutical composition according to claim 1, characterized in that it is in a form suitable for the oral administration.

3. Pharmaceutical composition according to claim 1, characterized in that it is in a form suitable for rectal administration.

4. Pharmaceutical composition according to claim 1, characterized in that it is in an injectable form.
